# EUROPEAN PATENT SPECIFICATION

(11) **EP 2 032 191 B1**
(45) Date of publication and mention of the grant of the patent: **12.08.2015**
(21) Application number: 07766838.2
(22) Date of filing: 28.06.2007
(51) Int. Cl.: A61M 1/30, A61B 17/88

(54) **INTEGRATED BONE BIOPSY AND THERAPY APPARATUS**
INTEGRIERTES KNOCHENBIOPSIE- UND THERAPIEGERÄT
BIOPSIE OSSEUSE INTÉGRÉE ET APPAREIL DE TRAITEMENT

(30) Priority: 29.06.2006 US 817070 P; 21.07.2006 US 832122 P
(43) Date of publication of application: 11.03.2009
(73) Proprietor: DePuy Spine, Inc., Raynham, MA 02767 (US)
(72) Inventor: GLOBERMAN, Oren, 46910 Kfar-shemaryahu (IL); BEYAR, Mordechay, 30889 Caesarea (IL)
(74) Representative: Orr, Robert
(86) International application number: PCT/IL2007/000808
(87) International publication number: WO 2008/001385

(56) References cited:
- EP-A1- 1 598 015
- US-A- 5 968 008
- US-A1- 2003 225 364
- US-A1- 2004 054 377
- US-A1- 2004 073 139
- US-A1- 2004 133 124
- US-A1- 2004 260 303
- US-A1- 2004 267 154
- US-A1- 2005 058 717

## Description

### FIELD AND BACKGROUND OF THE INVENTION

The present invention relates to apparatus for extracting a tissue sample from a body organ and injecting a therapeutic substance into that organ using a same cannula. Embodiments of the invention are particularly useful for extracting a sample of cancellous bone and injecting a bone cement or bone filler material into a bone.

Surgical and/or interventional treatment of fractured bones, osteoporotic bones, deformed bones and the like occasionally includes the use of various types of bone fillers, used to reinforce and stabilize the bone, restore its original configuration, and/or alleviate pain.

For example, vertebral fractures such as vertebral compression fractures (VCFs) may be treated using the vertebroplasty technique, during which a bone cement such as PMMA is injected into the vertebral body, usually through a cannula having a diameter of between 1 to 4 mm. Current vertebroplasty procedures typically rely upon a needle and stylet assembly.

One typical cause of vertebral fractures is existence of a malignant tumor. When suspicion of a malignancy exists, common clinical practice is to sample the suspected tissue by performing a biopsy prior to or during the VCF treatment. Known art in this field includes use of a working sleeve to penetrate into a vertebra (or other organ), introduction of a biopsy needle through the working sleeve into the bone area, withdrawal of needle and sleeve after tissue sampling, and then introducing an injection needle or cannula into the damaged vertebral body, and then using needle or cannula to deliver bone cement to reinforce the bone.

US Patent Publication No. US-2004/0267154 discloses a bone marrow sampling device including an outer cannula having a sidewall with a plurality of openings and a distal tissue penetrating tip. An inner cannula is positioned within the outer cannula, and includes a sidewall with an opening. Selective relative movement of the inner cannula and the outer cannula enables the device to be configured in multiple bone marrow sampling modes in which the opening in the sidewall of the inner cannula is aligned with different openings in the sidewall of the outer cannula, such that bone marrow can be drawn into the inner lumen of the inner cannula from different radial and longitudinal positions external to the sidewall of the outer cannula without the need to reposition the outer cannula.

European Patent Publication No. EP-1598015 discloses a localization fixture used in conjunction with a breast coil for breast compression, and for guiding a core biopsy instrument during prone biopsy procedures in both open and closed Magnetic Resonance Imaging (MRI) machines. The localization fixture includes a three-dimensional Cartesian positionable guide for supporting and orienting an MRI-compatible biopsy sleeve to a biopsy site of suspicious tissues or lesions. The sleeve receives a probe of the MRI-compatible biopsy instrument and may contain various features to enhance its imagability, to enhance vacuum and pressure assist therethrough, and marker deployment.

US Patent Publication No. US- 2005/0058717 discloses devices for delivering an orthopaedic cement to a target bone site in conjunction with vibration. Introducing a flowable cement to a target bone site in conjunction with vibration provides for controlled penetration of the delivered cement throughout the target bone site without the use of substantial pressure. The device comprises an aspiration cannula that extends through an entry cannula.

### SUMMARY OF THE INVENTION

The present invention provides an apparatus for extracting a biopsy sample and for injecting a therapeutic material as claimed in claim 1.

Further features of the apparatus are defined in the dependent claims.

Embodiments of the present invention provide apparatus for integration of biopsy sampling with VCF and vertebroplasty procedures, thereby potentially rendering such treatment more efficient and more rapid, saving time for surgeons and reducing risk to patients. Corresponding methods are also disclosed, but do not fall within the scope of the claimed invention. Embodiments of the invention provide cannula adapted both for extraction of an organic tissue sample from a body and also for injection of a therapeutic material into that body. Some embodiments can be used for collecting samples of cancellous bone tissue when the cannula is inserted in a bone, and then subsequently guiding a fluid material such as a bone cement into the same (or a nearby) tissue locus. Among embodiments for collecting samples of cancellous bone tissue include embodiments comprising a cannula having a side opening and an internal structure which guides a biopsy needle advanced through the cannula to pass through that side opening and thence to penetrate the bone tissue.

In some embodiments, the cannula comprises a plurality of slits, which are provided to facilitate plastic deformation of the cannula. In some cases the slits are sized and positioned in such a way that when the cannula is bent in a pre-planned direction and to a pre-planned extent, slits which were initially somewhat open are closed by the bending, so that although the slits provide enhanced flexibility of the cannula as a whole, there is little or no tendency of fluid traversing through the bent cannula to leak therefrom. In particular, the size, shape, and position of the slits may be so calculated that when the cannula is bent in a predetermined direction, the amount of opening in the cannula is reduced, as compared to the slit openings in the cannula when the cannula is unbent.

Since it is anticipated that the cannula will often be used in a clinical context where imaging modalities such as x-ray imaging and in particular fluoroscopy will be used to guide placement of the cannula and to monitor cannula use, in some embodiments a manipulating rod is provided, together with a connector for connecting rod to cannula, such that with rod connected to cannula, a surgeon can manipulate and control the cannula by holding the rod. The manipulating rod is preferably sufficiently long to enable a surgeon to manipulate the cannula while at least the distal portion of the cannula is inserted in a body of a patient and is illuminated by x-ray or other energy sources emitted by imaging modalities, without the surgeon's hands thereby being exposed to the illuminating radiation. In general, the manipulating rod connectable to the cannula is sufficiently long so that a distal portion of the rod protrudes beyond an irradiated field when a treatment target is irradiated by an irradiating imaging modality.

In some embodiments the apparatus further comprises a stylet advancable and retractable within the cannula. The stylet may be rigid, and serve to straighten and strengthen the cannula, particularly during insertion of the cannula into hard body tissues. Alternatively, the stylet may impose a predetermined curvature to the cannula.

In some embodiments the stylet comprises a cutting tip.

In some embodiments the stylet is designed and constructed to be distinguishable from the cannula under various imaging modalities. For example, the radio opacity of the stylet or of the stylet tip may be made to differ from the from radio opacity of the cannula, so that the stylet tip extending beyond a distal end of the cannula, or extending from a lateral opening in the cannula, can be clearly distinguished on an x-ray or fluoroscopic image. Similarly, the stylet may be designed so that a difference in diameter of the cannula as compared to the diameter of the stylet is sufficient to be visible on an x-ray or other image.

A surface feature of the stylet (such as a bevel) and a corresponding surface feature of an internal lumen of the cannula may be provided, to constrain the stylet to enter and traverse the cannula in a predetermined orientation.

In some embodiments two distinct handles are provided, a first proximal handle connected to the cannula and a second proximate handle connected to the stylet, the two handles enabling a physician to differentiate forces applied to the cannula from forces applied to the stylet. In one possible use of this two-handle configuration, matching orientation of one of the handles to orientation of cannula slits is used to facilitate bending of the cannula, so that in typical use, the forces applied by a surgeon to insert the cannula will not be in the same direction as forces applied by the surgeon to bend the cannula.

In some embodiments, the cannula has a side opening and the stylet has a lateral niche. Opening and niche may be sized and oriented (or orientable) in such a way that at some positions of the stylet within the cannula at least a portion of said lateral niche is aligned with the cannula's side opening. This aligned position may be used during the biopsy sampling process, in that the niche may serve as trap area for entrapping tissues or other materials or substances that enter the cannula through the side opening. Subsequent to such entrapment of such tissues substances, the stylet may be retracted from the cannula, bringing with it the entrapped materials, which can then be examined in a clinical laboratory for diagnostic or other purposes. In a recommended mode of use, when the stylet is removed for the cannula, the cannula is not itself retracted from the body, but rather remains in place within the body with its distal end positioned at or near the biopsy sampling site. A proximal end of the cannula is then connected to a fluid source. The fluid source, once connected to a proximal portion of the cannula, may then be used to force a fluid under pressure through the cannula and into the body tissues. In one embodiment, the fluid source is a reservoir of a bone cement having a consistency which enables it to flow through the cannula and into the bone.

In some embodiments, the cannula has a side opening and the apparatus further comprises a biopsy needle bendable at least on its distal end. In this embodiment cannula, the internal lumen of the cannula leading to a side opening is slanted or curved near the opening, so that a biopsy needle advanced through the cannula is forced to curve sideways and to protrude out through the side opening of the cannula, where it may be forced into body tissues to take a tissue sample. Subsequent to the taking of the sample, the biopsy needle can be retracted from the body tissue and further retracted through the cannula, after which a fluid source such as a reservoir of a flowable bone cement or bone-filling material, or other therapeutic substance, may be connected to a proximal portion of the cannula, enabling to flow the bone cement (or other therapeutic fluid) through the cannula and into a region at or near the site from which the tissue sample was taken.

The fluid source connectable to the cannula may be a simple reservoir of fluid which may be forced to advance through the cannula by means of manual pressure through the reservoir. Alternatively, a pump or impeller or other pressure source may be supplied to inject a flowable material through the cannula and into the body. If a highly viscous material is being supplied, a vibrator may be provided to vibrate portions of the apparatus and thereby to facilitate flow of that viscous material through portions of the apparatus.

In some embodiments the cannula is designed and constructed in a manner which facilitates imposing a planned plastic deformation, such as bending, to a selected portion of the cannula, during a surgical procedure. For example, in some embodiments the cannula comprises a series of slits designed to facilitate the imparting of a plastic deformation to a specific portion of the cannula. The slits may be so shaped, sized, and positioned that when the cannula has undergone a planned plastic deformation, such as bending to a planned degree, the bend of the cannula substantially closes the slits, thereby limiting or preventing leaking of fluid from within the cannula outward though the slits.

### BRIEF DESCRIPTION OF DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
Fig. 1A is a simplified schematic of an apparatus for tissue sample extraction and for injection of a flowable material at or near the sampling site, according to an embodiment of the present invention;
Fig. 1B is a simplified schematic of a biopsy needle penetration limitation module, according to an embodiment of the present invention;
Fig. 2A is a simplified schematic of a cannula with a side opening, according to an embodiment of the present invention;
Fig. 2B is a detailed view of a portion of the cannula of Figure 2A, according to an embodiment of the present invention;
Fig. 2C presents the cannula of Figure 2A with an optional closed beveled tip, according to an embodiment of the present invention;
Fig. 3A is a simplified schematic of a stylet with a lateral niche for use as a tissue extractor, according to an embodiment of the present invention;
Fig. 3B is a detailed view of a portion of the stylet of Figure 3A, showing the lateral niche, according to an embodiment of the present invention;
Figs. 4A, 4B and 4C are simplified schematics showing a process by which a sampling of cancellous bone tissue may be obtained, utilizing an apparatus which comprises a cannula and a stylet, according to an embodiment of the present invention;
Figs. 4D, 4E, 4F and 4G are simplified schematics illustrating a process by which a cannula, stylet, and biopsy needle assembly can be used to obtain a sample of cancellous bone tissue, according to an embodiment of the present invention.
Fig. 5A is a simplified perspective view of an elongated rod attached to a cannula, according to an embodiment of the present invention;
Fig. 5B is a simplified view of an optional distal end of an elongated rod attachable to a cannula, having a triple-teeth interlocking mechanism, according to an embodiment of the present invention;
Fig. 5C is a simplified view of an optional distal end of an elongated rod attachable to a cannula, the rod end comprising an interlocking mechanism which incorporates frontally pressed snaps, according to an embodiment of the present invention;
Fig. 5D is a simplified view of an optional distal end of an elongated rod attachable to a cannula, the rod end comprising an interlocking mechanism which incorporates laterally pressed snaps, according to an embodiment of the present invention;
Fig. 6 is a simplified schematic of a deformable cannula with stylet inserted, according to an embodiment of the present invention;
Fig. 7A is a simplified schematic of a deformable cannula prior to deformation, according to an embodiment of the present invention;
Fig. 7B is a detailed view of a portion of the cannula of Figure 7A, showing in detail the cannula slits, according to an embodiment of the present invention;
Fig. 8A and 8B present views of the cannula of Figures 7A and 7B, showing the cannula form subsequent to deformation, according to an embodiment of the present invention;
Fig. 8C is simplified schematic of cannula slits similar to those presented in Figures 7 and 8, showing slit shapes in detail, according to an embodiment of the present invention; and
Fig. 9 is a simplified flow diagram of a method for providing a therapeutic substance, such as bone filler material, to a body organ such as a bone, with optional biopsy sampling, according to an embodiment of the present invention.

### DESCRIPTION OF THE PREFERRED EMBODIMENTS

The following describes apparatus and methods for utilizing a cannula having a distal portion insertable into a body, both to obtain body tissue samples and to deliver a therapeutic material to a target site within that body. Specifically, the present invention can be used to obtain a sample of cancellous bone tissue and to deliver bone cement or another therapeutic material to a site on or within a bone.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

For exemplary purposes, the present invention is principally described in the following with reference to an exemplary context, namely that of intervention within a bone, sampling of cancellous bone tissue, and injection of a bone cement into the bone structure. It is to be understood that the invention is not limited to this exemplary context. The invention is, in general, relevant to use of a cannula or any similar device to both extract a tissue sample from an intervention site and to supply a flowable therapeutic material at or near that intervention site. For simplicity of exposition, interventions in bony structures are presented in the Figures and throughout the discussion hereinbelow, yet all such references are to be understood to be exemplary and not limiting. Thus, discussion hereinbelow may be understood to apply also to cannulas or similar tools having forms different from those presented in the Figures, including cannulas presented within or combined with other surgical tools. References to biopsy sampling of bone tissue and therapy of bony structures are to be understood as referring also to biopsy sampling and treatment of non-bony sites, and references to providing or injecting of bone filling material or bone cement may be understood to refer also to the supplying of therapeutic materials of other sorts to a site within a body organ.

It is expected that during the life of this patent many relevant forms of cannula, stylet, and biopsy needle will be developed, and the scope of the terms "cannula" and "stylet" and "biopsy needle" is intended to include all such new technologies a priori.

In discussion of the various figures described hereinbelow, like numbers refer to like parts. The drawings are generally not to scale. Some optional or non-essential parts may be omitted.

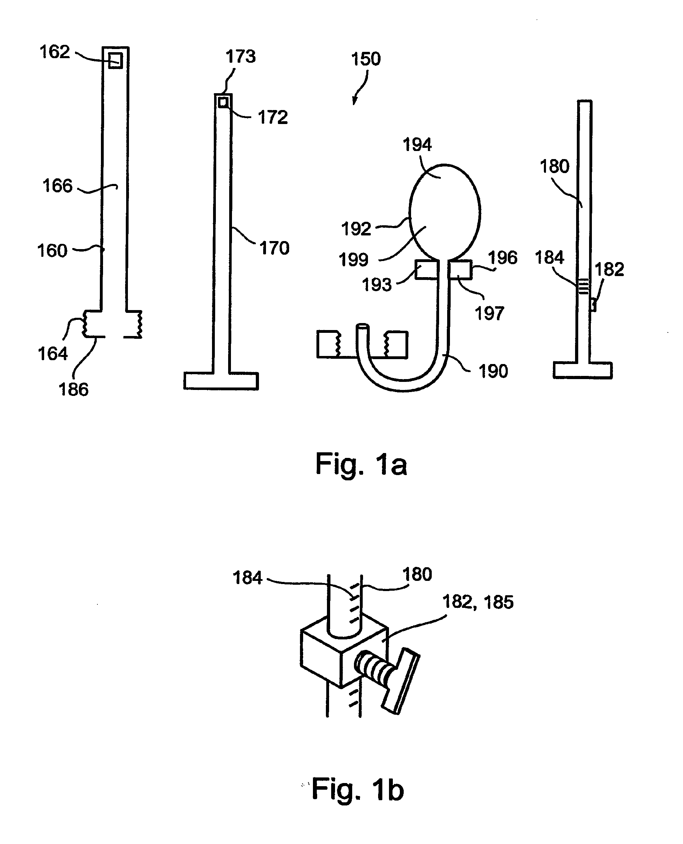

Attention is now drawn to Figure 1A, which is a simplified schematic of an apparatus for removal of a biopsy sample from a treatment site and for injection of a therapeutic material into that site.

Figure 1A presents apparatus 150 which comprises a cannula 160, a stylet 170 and/or biopsy needle 180, and a fluid source 190. Fluid source 190 is also referred to herein as injector 190 and as therapeutic material supply 190. Apparatus 150 is optionally supplied as a kit containing the components shown in Figure 1A; the components sized to fit optimally together, connectors and fittings adapted one to another, etc.

Cannula 160 comprises an internal lumen 166 and a distal opening 162, and preferably comprises a proximal connector 164.

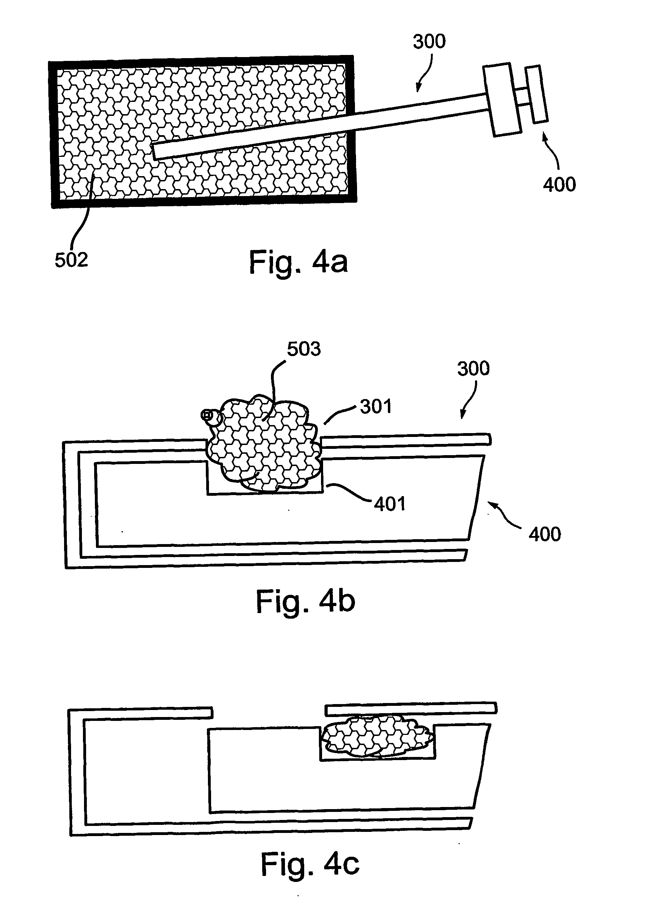

Stylet 170 is sized to fit within lumen 166 and to be advancable and retractable within lumen 166. Distal end 173 of stylet 170 may optionally be sharpened or hardened. Stylet 170 may comprise a distal niche or recess 172, which is so sized and oriented as to enable niche 172 to be at least partially aligned with opening 162 at certain positions of stylet 170 within lumen 166. As will be discussed in detail hereinbelow, stylet 170 may be inserted in cannula 160 to straighten and strengthen cannula 160 while a distal portion of cannula 160 is caused to penetrate into a body and optionally into a bone. Once distal opening 172 is positioned at a desired target locus, stylet 172 may be used to remove a tissue sample from that target locus, as will be explained in detail below. Alternatively, a biopsy needle 180, also sized to fit and operable to be advanced and retracted within lumen 166, may be used to take a tissue sample at the target locus. Biopsy needle 180 may be advanced through cannula 160 until its distal end protrudes through distal opening 162, for taking of bone samples or other tissue samples. Distal opening 162 may be a side opening, and the distal end of biopsy needle 180 may be caused to project laterally from cannula 160 as it passes through opening 162 embodied as a side opening. Details of several exemplary embodiments of these configurations will be described in detail hereinbelow, in particularly with respect to Figures 4A-4G.

Apparatus 150 may comprise a penetration limitation module 182, provided to enable accurate pre-determination of a distance by which biopsy needle 180 will project through distal opening 162.

Attention is now drawn to Figure 1B, which is a simplified schematic of an exemplary embodiment of penetration limitation module 182, according to an embodiment of the present invention. Figure 1B shows a portion of biopsy needle 180 with a position-adjustable embodiment 185 of penetration limitation module 182 positioned thereon. As may be seen from Figure 1B, a simple screw mechanism enables to fix module 185 at a selected position on a proximal portion of biopsy needle 180. When biopsy needle 180 is inserted in cannula 160, penetration limitation module 182 will come up against proximal base 186 of cannula 160 (shown in Figure 1A), preventing further penetration. If module 182 is positioned appropriately on needle 180, forward motion of needle 180 will be stopped at a point where a desired length of distal portion of needle 180 is extended through distal opening 162. Graduated distance markers 184 may be provided on the shaft of needle 180 to facilitate positioning of module 182 and appropriate positions on needle 180, markers 184 being calibrated to show distances by which a distal end of needle 180 will extend beyond distal opening 162 of cannula 160 at various settings (positions) of module 182.

Alternatively, module 182 may be implemented in non-adjustable fashion. Module 182 may, for example, simply be a bump on the shaft of needle 180 or a handle attached thereto, preventing penetration of needle 180 into cannula 160 beyond a pre-determined point. When apparatus 150 is supplied as a kit, a plurality of biopsy needles 180 may be supplied, each designed with a fixed module 182, these modules 182 being at various distances from a distal end of the needles, thereby providing a user with a selection of biopsy needles each with a pre-determined penetration limitation. In general, modules 182 are useful in enabling biopsy samples to be taken from pre-determined depths within a bone or other tissue.

Returning attention now to Figure 1A, once a tissue sample has been taken, stylet 170 and/or biopsy needle 180 may be removed from within cannula 160, and a fluid source 190 may be attached to a proximal end of cannula 160 and used to flow a material into cannula 160 and through cannula 160 into the target locus. Fluid source 190 may be a simple material reservoir 192 made of compressible material, such that manual pressure on reservoir 192 causes a therapeutic material 194 contained therein to flow into cannula 160 under pressure. Alternatively, material source 190 may be provided with a pressure source 196, such as a pump 197, which serves to cause material from reservoir 192 to flow into cannula 160 under pressure. A vibrator 193 may also be provided to facilitate movement of viscous material through the apparatus. Vibrator 193 may be attached to cannula or to fluid source 190. It is to be understood that "fluid source 190" may supply a material which is more or less fluid: in some cases "fluid source 190" may supply a material which is highly viscous or nearly solidified, and which may be caused to flow through cannula 160 only under very high pressures. Material supplied by fluid source 190 is also referred to herein as "flowable material", indicating that, whatever its consistency, it is operable to be advanced through a catheter and into a body by means of pressure supplied by "fluid source 190."

As stated above, pressure source 196 may be designed to supply extremely high pressures. It is noted that supply of high pressures by pressure source 196 provides an additional advantage, in that high-pressure supply of flowable material through cannula 160 enables cannula 160 to be of very narrow dimension. By way of comparison, prior art biopsy needles are typically about 2 mm in diameter, but cannulas used to deliver bone cement or similar materials to a bone or similar site are typically 4 mm in diameter. In contrast, in some embodiments cannula 160 has a diameter of less than 3 mm. In an exemplary embodiment, cannula 160 is 2.3 mm in diameter, and a high-pressure is a high-pressure source 196 is provided.

Material supplied from source 190 will advance through cannula 160 to its distal end, and exit opening 162 into the target locus. Apparatus 150 may thus be used, for example, to take a sample of cancellous bone tissue, and then to inject a bone cement or other therapeutic material into a lesion or other therapeutic target. Source 190 may comprise a connector 191 adapted to connect to proximal connector 164 of cannula 160, to facilitate transfer under pressure of material from supply 190 to cannula 160.

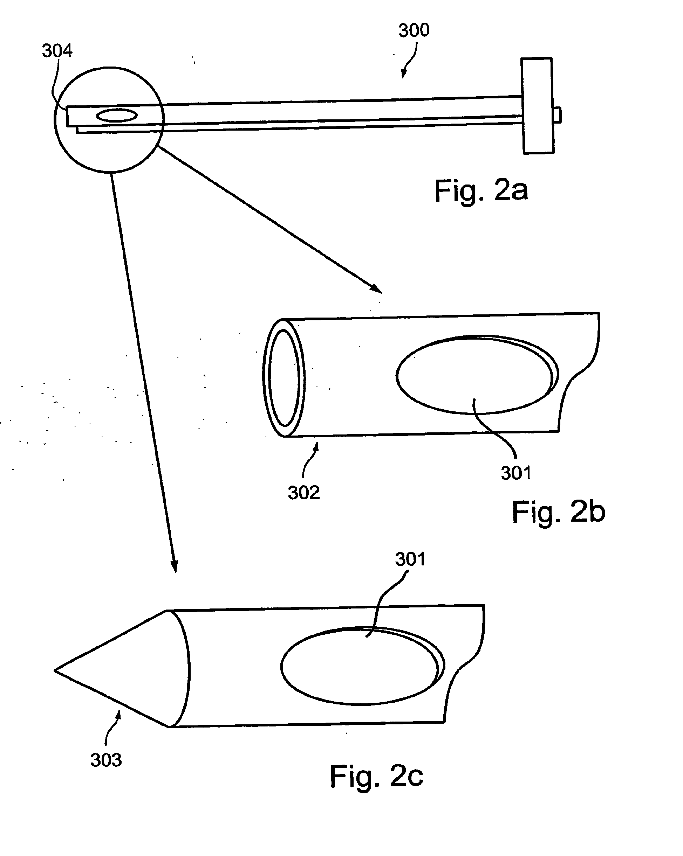

Attention is now drawn to Figures 2A, 2B and 2C, which are simplified schematics of a cannula, according to an embodiment of the present invention. Figures 2A, 2B and 2C show views of a cannula 300 with a side opening 301. Cannula 300 is thus an embodiment of cannula 150 presented in Figure 1A. Side opening 301 may serve as an intake and/or an outlet port for materials and/or instruments. Optionally, side opening 301 may serve as an injection port for bone filler material to be delivered into a body organ through cannula 300. Optionally, side opening 301 may serve as an intake port for biopsy sampling of living tissues, e.g. cancellous bones.

Distal tip 304 of cannula 300 may be open, allowing materials to pass through tip 304 in and out of cannula 300, or may be closed, preventing passage of materials. Figure 2B illustrates an optional distal end pattern of cannula 300, having a distal tip 302 which is open and flat. Figure 2C illustrates another optional distal end pattern of cannula 300, having a distal tip 303 which is closed and shaped as a cone. In general, distal tip 304 may be of any shape or pattern. Optionally, tip 304 may be sharpened, be hardened, be provided with drilling surfaces or be otherwise adapted to facilitate penetration of cannula 300 into or through hard tissue such as bone. Alternatively, when cannula 300 is used together with a stylet such as stylet 170, distal tip 173 of stylet 170 may be sharpened, hardened, may be provided with drilling surfaces or may be otherwise adapted to facilitate penetration of hard tissues. In this case, tip 173 is optionally pushed forward so that it extends beyond tip 304 and serves as a penetration tool to facilitate penetrate of cannula 300 into bone or other hard tissue. In general, one or more than one lateral ports or distal ports may be provided, and a cannula may optionally be provided with a tip 304 designed to facilitate penetration of tissues.

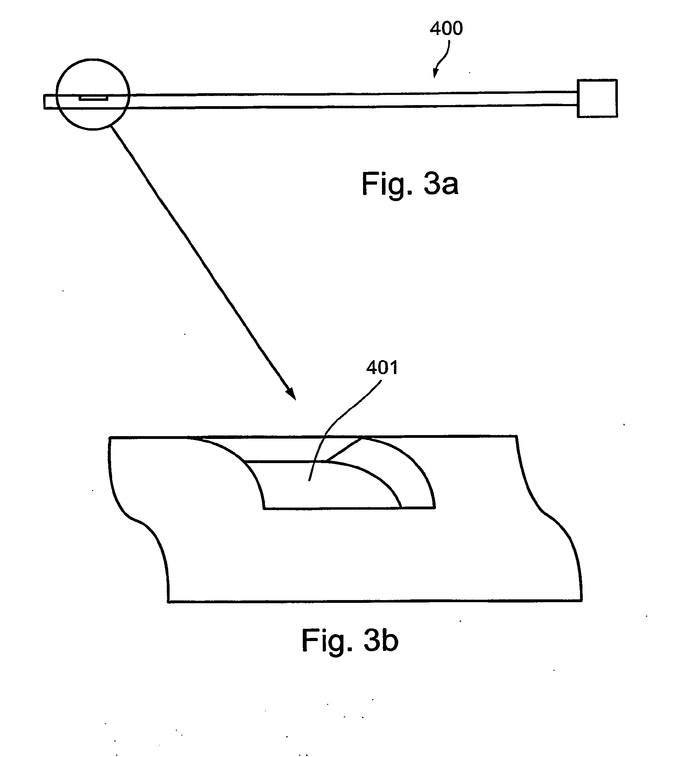

Attention is now drawn to Figures 3A and 3B, which are simplified schematics of a stylet such as stylet 160 or similar stylet, for use with a cannula 150 or 300 or similar cannula, according to an embodiment of the present invention.

Figures 3A and 3B illustrate an exemplary embodiment, here labeled stylet 400, having a lateral niche 401. Optionally, stylet 400 is sized to fit into cannula 300, or a similar cannula having a side opening.

Preferably, stylet 400 can be positioned within cannula 300 in such position that side opening 301 at least partially faces lateral niche 401. Such placement enables lateral niche 401 to serve as trap area for entrapping materials or substances that enter into cannula 300 through side opening 301. An apparatus (such as apparatus 150) comprising a cannula 300 and a stylet 400, appropriately sized and configured one with respect to the other, can be used to collect samples of living tissues (e.g., cancellous bones) for biopsy evaluation.

Attention is now drawn to Figures 4A, 4B, and 4C, which are simplified schematics showing stages in use of apparatus 150 (or a similar apparatus) to take a tissue samples, according to an embodiment of the present invention.

Figure 4A shows an apparatus combining a cannula 300 having a side opening 301 and a stylet 400 having a lateral niche 401 being used to access and/or move through a portion of cancellous bone 502. When lateral niche 401 is at least partially aligned with side opening 301, as illustrated in Figure 4B, a sample 503 of cancellous bone 502 may enter lateral niche 401 through side opening 301. Stylet 400 may then be partially withdrawn from cannula 300, thereby entrapping at least a portion of sample 503 between niche 401 and an outside wall of cannula 300, as shown in Figure 4C. Stylet 400 may then be wholly withdrawn from cannula 300, making sample 503 accessible to appropriate clinical analyses for purposes of evaluating the health or pathology of tissues 502. Thus, Stylet 400 functions as a tissue extractor advancable and retractable within cannula 300. It is noted that movement of niche 401 with respect to opening 301 may optionally be coordinated with both longitudinal and axial movement of cannula 300, to facilitate capture of a tissue sample from a desired locus.

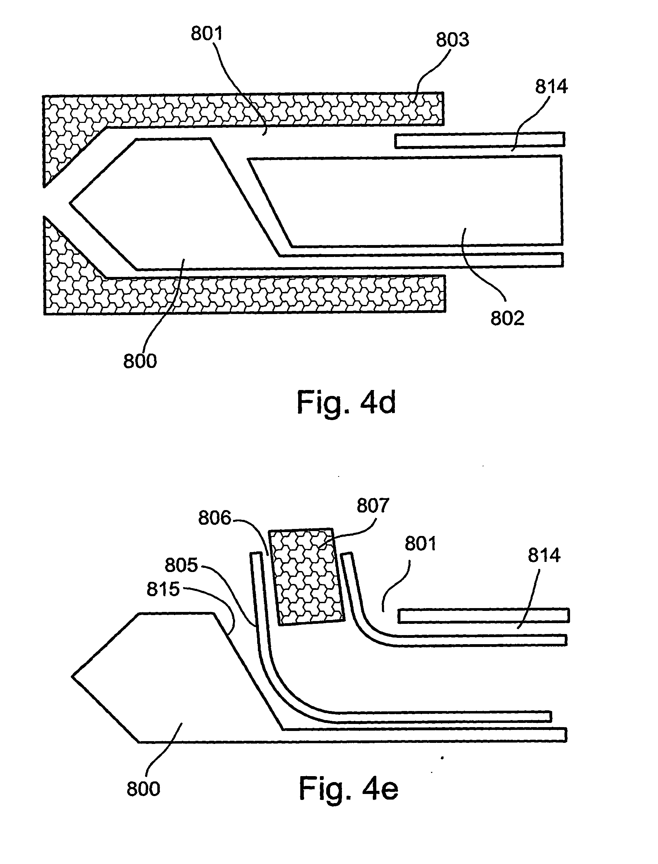

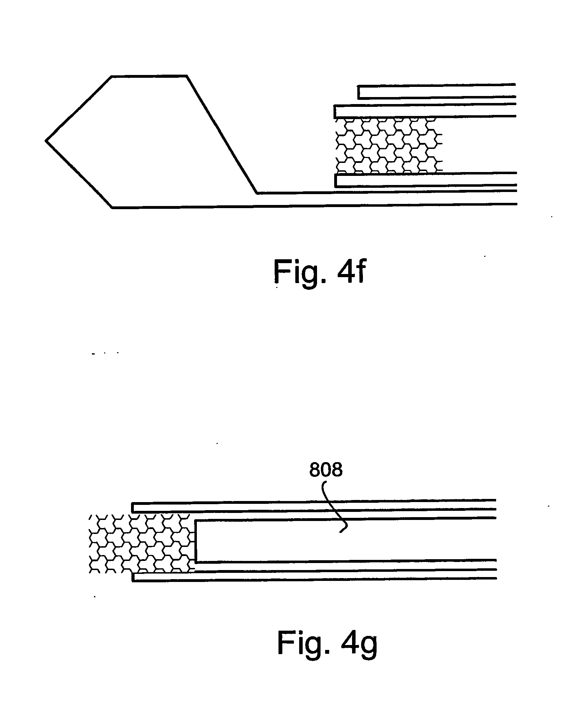

Attention is now drawn to Figures 4D, 4E, 4F, and 4G, which are simplified schematics presenting a method for sampling cancellous bone tissue, which method uses a bendable biopsy needle as a tissue extractor operable to sample bone or other tissue by extending laterally from a cannula, according to an embodiment of the present invention.

Figure 4D shows a cannula 800 having a side opening 801 and internal lumen 814 and an optional stylet 802 used to access and/or move through a portion of cancellous bone 803. Stylet 802 serves to cover side opening 801 and/or to stiffen cannula 800 during insertion of cannula 800 into a target locus within bone volume 803. Once cannula 800 is inserted into a desired locus, stylet 802 may be withdrawn. As shown in Figure 4E, a biopsy needle 805 may then be inserted into cannula 800 and through cannula 800 to side opening 801. As illustrated in Figure 4E, biopsy needle 805 has a flexible or bendable distal end, and distal end 815 of internal lumen 814 of cannula 800 has a slanted or curved shape, which shape forces the distal end of biopsy needle 805 towards and through side opening 801 if needle 805 is pushed into cannula 800 to end 815 with sufficient force. In this manner biopsy needle 805 can be forced to advance, through side opening 801, into cancellous bone 803. A cancellous bone sample 807 will be entrapped, by this procedure, in distal opening 806 of biopsy needle 805, which is preferably sufficiently strong to withstand being pressed to advance into cancellous bone, yet comprise a portion sufficiently flexible to bend as shown in Figure 4E.

As illustrated in Figure 4F, biopsy needle 805 may then be withdrawn from cannula 800, with at least part of sample 807 trapped within its distal portion. Sample 807 may then be pushed out of biopsy needle 805, for example by pressure from an inner rod 808 inserted into biopsy needle 805 from its proximal end, as illustrated in Figure 4G. Sample 807 is thus made available for pathological evaluation.

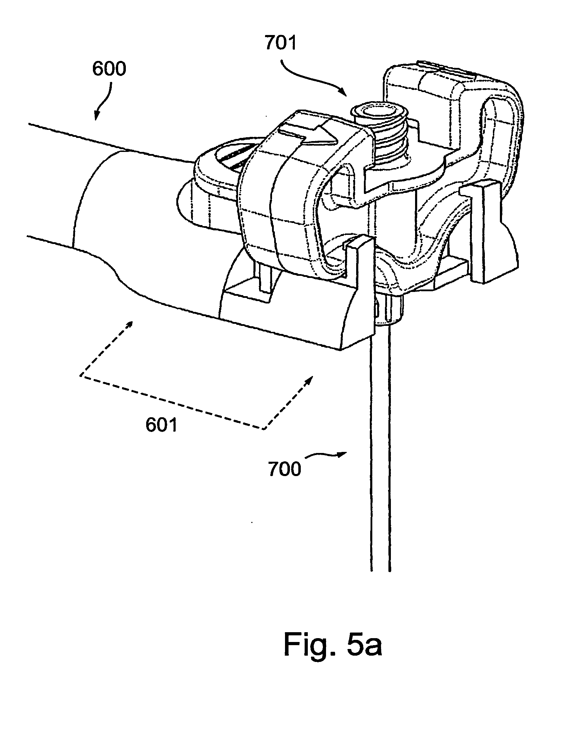

Attention is now drawn to Figure 5A, which is a simplified view of a manipulating rod used to manipulate a cannula, according to an embodiment of the present invention. Figure 5A presents a perspective view of a distal side of an elongated rod 600 interlocked to a handle 701 of a cannula 700. Rod 600 is attached to cannula 700 by a locking mechanism 601. In clinical contexts in which bone samples are taken and bones are treated, it is common practice to monitor surgical procedures by use of imaging modalities such as x-ray and in particular fluoroscopy. Rod 600 is preferably sufficiently long so when that when rod 600 is interlocked with (i.e. attached to) a cannula and/or any other bone surgery instrument, the hands of an operator holding rod 600 and thereby enabled to hold, control, and manipulate the cannula or related tool, while remaining substantially or entirely out of the field of radiation projected by the imaging modality. A desirable length for rod 600 will depend on a specific use (e.g. a specific site being treated within a body) for which a given embodiment is intended. In an exemplary embodiment, rod 600 is 20 cm in length, yet longer and shorter lengths may be useful in particular clinical contexts.

Rod 600 is may be re-usable or designed for one-time use.

Interlocking mechanism 601 is preferably capable of sustaining a rigid connection between elongated rod 600 and cannula 700, so that cannula 700 can be held and manipulated as a bone access instrument and biopsy sampler and/or as a filler material delivery device, by a user holding and manipulating rod 600.

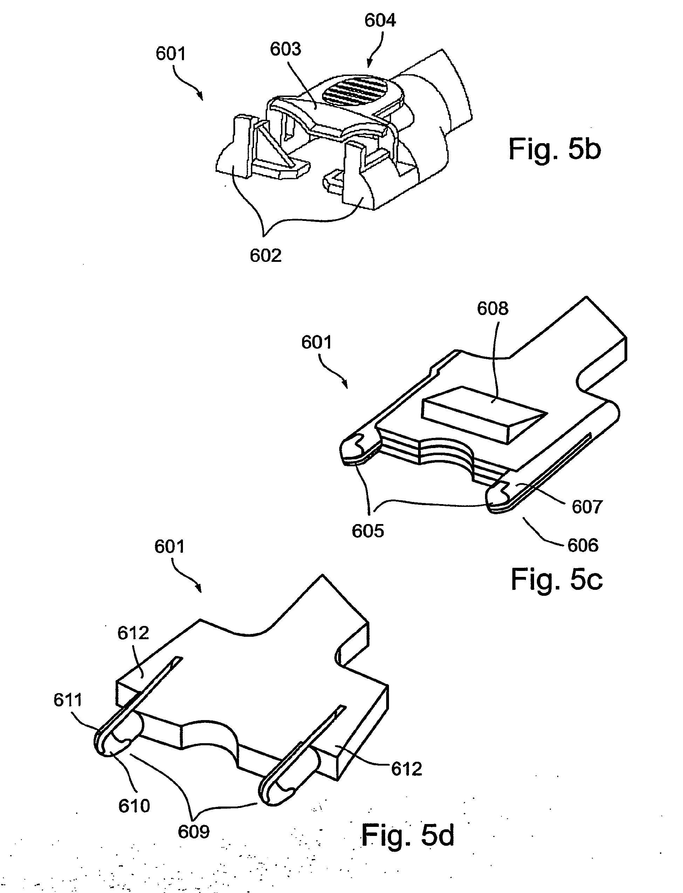

Attention is now drawn to Figures 5B, 5C and 5D, which are simplified perspective views of exemplary alternative constructions of interlocking mechanism 601, according to embodiments of the present invention. Figure 5B shows an interlocking mechanism 601 having a combination of rear tooth 603 and front teeth 602. Optionally, front teeth 602 are rigidly fixed to interlocking mechanism 601, while rear tooth 603 may be shifted by pressing or releasing button 604. This combination of teeth may be used to hold between them a cannula handle 701. When button 604 is pressed (or when it is released), interlocking mechanism 601 is rigidly connected to handle 701, and when button 604 is released (or when it is pressed) these elements are disconnected.

Figure 5C illustrates a second example of an interlocking mechanism 601, one which comprises frontally pressed snaps 605. Preferably, each snap 605 consists of a flexible bottom leg 606 and a flexible upper leg 607, separated by an elongated space, so when both legs are pressed one to the other, snap 605 collapses to a smaller diameter; and when released, snap 605 re-expands to its original dimensions. Optionally, legs 606 and 607 are pressed together by pressing a button 608. This interlocking mechanism may be used to hold a cannula handle 701, so when button 608 is released snaps 605 may be clicked into matching holes in handle 701. Preferably, these holes have a diameter smaller than that of snap 605 in its expanded form and larger than that of snap 605 in its collapsed form, so interlocking mechanism 601 can be interlocked with or released from handle 701 by using button 608.

Figure 5D illustrates a third example of interlocking mechanism 601, one which comprises laterally pressed snaps 609. In a mechanism similar to that described above with respect to figure 5C, each snap consists of a flexible right leg 610 and a flexible left leg 611, which legs may be laterally pressed one to the other by pressing opposing areas 612, thus providing an additional interlocking mechanism 601 which may also be used to hold a cannula handle 701 in a rigid connection. In general, rod 600 may be attached to cannula 700 with any variety of snaps, interlocking teeth, screw mechanism, clamp mechanism, or any other connective device which provides a rigid or semi-rigid connection.

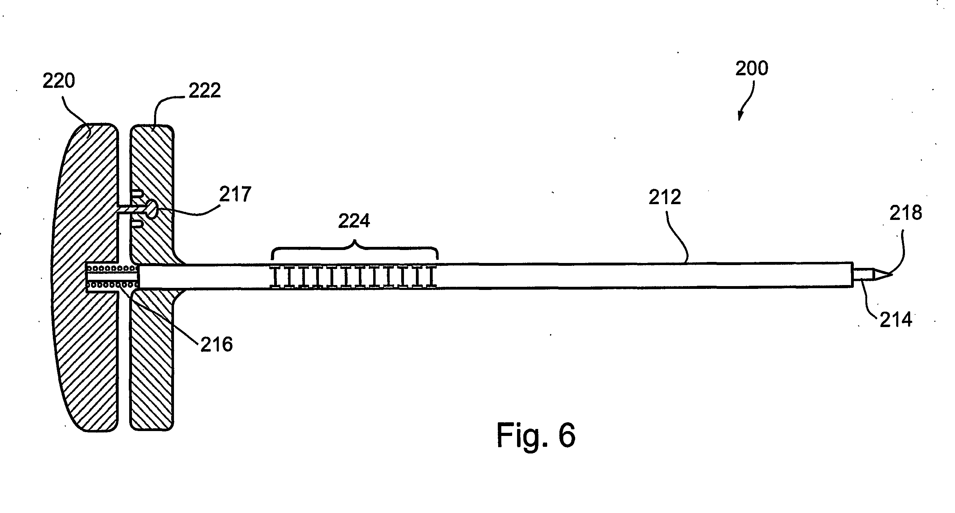

Attention is now drawn to Figure 6, which is a simplified schematic of an apparatus comprising a cannula with a first handle and a stylet with a second handle, according to an embodiment of the present invention. Figure 6 presents a stylet 214 having a handle 220. Stylet 214 is inserted within a cannula 212 having a handle 222. Cannula 212 may be an embodiment of cannula 160 and/or of cannula 300, both described above, and stylet 214 may be an embodiment of stylet 170 and/or of stylet 400, both also described above.

Cannula 212 comprises a series of slits 224 provided to impart a desired plastic deformation capability to a specific portion of cannula 212.

Stylet 214 is inserted through cannula 212 via an inner lumen thereof (corresponding to lumen 166 of Figure 1A). A cutting tip 218 of stylet 214 optionally protrudes from a distal end of cannula 212 when stylet 214 is fully inserted therein. Tip 218 is optionally adapted to puncture and penetrate skin, soft tissue, cortical bone and/or other bone. Tip 218 may be, for example, of diamond type, drill type, bevel type or J-type, or of other tip types known in the art.

Optionally, cannula 212 and stylet 214 are so constructed that their respective distal tips are visibly different from one another when observed under clinical imaging modalities. For example, they may have different degrees of radio-opacity, or discernibly different diameters, or have some other observable difference serving to make them distinguishable, so as to enable a viewer to distinguish one from another when both are viewed by means of an imaging modality such as a fluoroscope.

An internal lumen of cannula 212 is adapted to receive stylet 214. For example, an inner cannula lumen of diameter 2.7 mm may be provided with a stylet of external diameter 2.6 mm. Stylet 214 and the internal lumen of cannula may have surface features (e.g. a bevel) which constrain stylet 214 to enter cannula 212 in a predetermined orientation. Such a feature is useful, for example, in a context such as that presented by Figures 4A-4C and discussed hereinabove, where it is important that there be a position at which side opening 301 at least partially faces lateral niche 401.

Optionally, stylet 214 is equipped with a proximal handle 220. In some embodiments of the invention, handles 222 and 220 engage one another via an engagement mechanism 216, which may be, for example, a threaded connection. Optionally, a spring is provided to elastically couple the components. An alternative locking mechanism 217 is also shown in Figure 6. In locking mechanism 217 a tongue on one handle snap-locks to a groove on the other handle. Such snap-locking may be, for example, by rotation or by axial motion.

Handle orientation may be matched to slit orientation (described below), so that in typical use, forces applied by a doctor to insert cannula 212 into a body will not be in the same direction as forces that are used to bend cannula 212. Handle direction may be used to indicate the desired deformation direction.

In some embodiments of the invention, stylet 214 is rigid. A rigid stylet 214 supports cannula 212 during insertion and prevents deformation of cannula 212 until such deformation is desired. Stylet 214 may then be removed before deformation is undertaken.

In alternative embodiments, stylet 214 may be curved.

Stylet 214 may be flexible, or a portion of stylet 214 may be flexible, for example a portion positioned to correspond to slit series 224 when stylet 214 is inserted in cannula 212.

In an exemplary embodiment of the invention designed for use in a fractured vertebral body, stylet 214 has a diameter of about 1.4-2.6 mm. The cannula optionally has an inner diameter of about 2.7 mm and an outer diameter of about 3 mm. When employed in a vertebroplasty procedure, the assembled cannula stylet 200 is introduced into the body so that distal tip 218 penetrates skin, soft tissue and vertebra. Stylet 214 is then disconnected from cannula 212, which remains in situ during delivery of a therapeutic material to the vertebra, as will be described in detail hereinbelow.

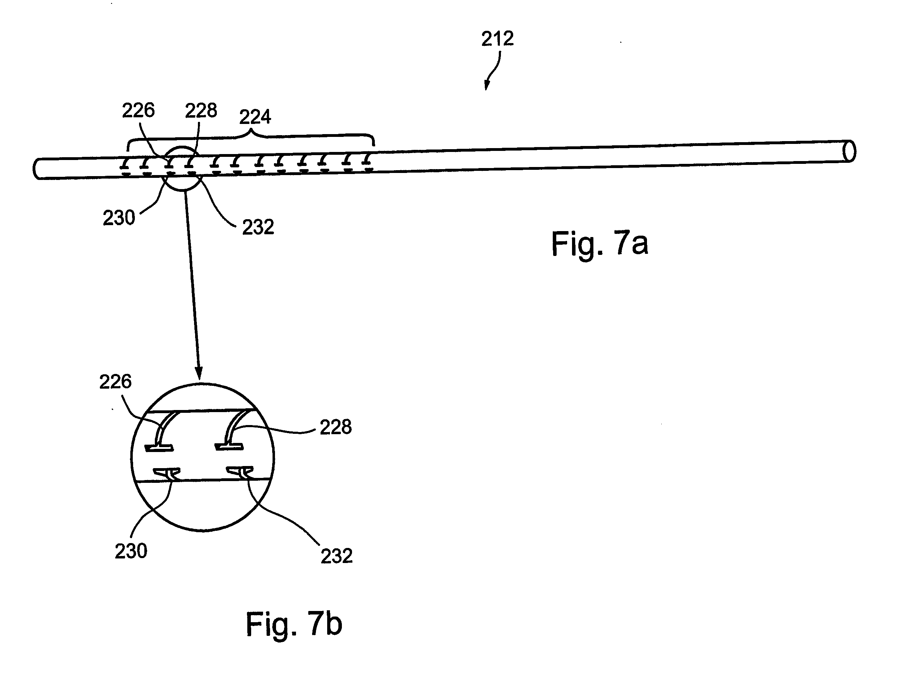

Attention is now drawn to Figures 7A and 7B, which are detailed views of cannula 212, according to an embodiment of the present invention. Figure 7A presents cannula 212 with stylet 214 removed. The straight configuration of cannula 212, shown in Figure 7A, may be preferable for use during introduction of cannula 212 into a treatment site within a body. Cannula 212 comprises a series of slits 224, which slits facilitate plastic deformation (bending) of cannula 212.

Figure 7B (inset) is an enlargement of a portion of series of slits 224, showing several slits in greater detail. The pattern of slits shown in enlarged Figure 7B includes two rows of slits, 226, 228, etc., and 230, 232, etc. The rows are displaced 180 degrees relative to each other on the circumference of cannula 212. In an exemplary embodiment shown in Figure 7A, each row comprises 12 slits, but this number is exemplary only; other numbers of slits may be provided. Slits 224 may penetrate the cannula wall completely, or they may be perforations or grooves etched in the cannula wall.

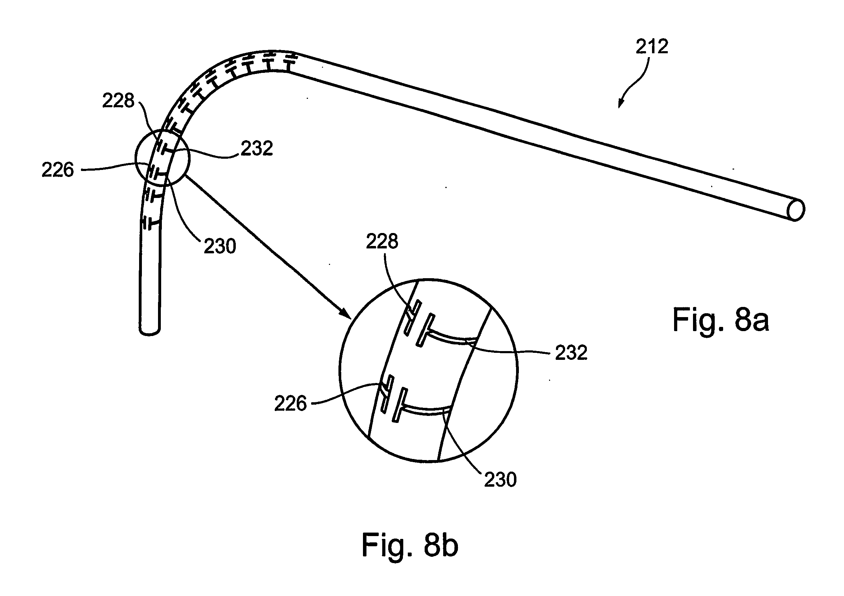

Attention is now drawn to Figures 8A and 8B, which show cannula 212 of Figures 7A and 7B after plastic deformation. As seen most clearly in Figure 8B, slits 230 and 232, on the inner side of the bend caused by the deformation, tend to close as a result of the deformation, and slits 226 and 228, on the outer side of the bend caused by the deformation, tend to remain of unchanged size, or tend to open slightly, as a result of the deformation.

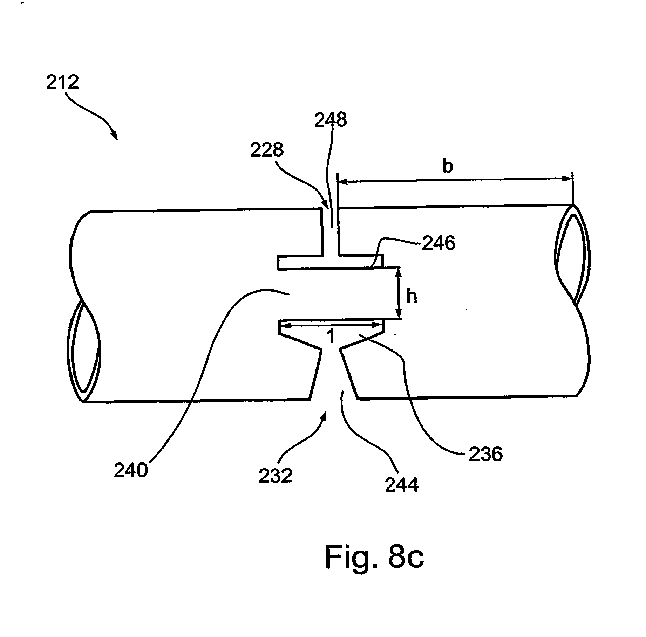

Attention is now drawn to Figure 8C, which is a detailed view of a pair of slits within a cannula wall, according to an embodiment of the present invention. Opening and closing of slits under plastic deformation of cannula 212 may understood with reference to Figure 8C. If the portion of cannula 212 shown in Figure 8C is caused to curve downward (in the sense of the Figure, that is, the ends of the shown cannula portion move downward and the center of the shown cannula portion moves upward), then opening 248 of slit 228 will tend to widen, and opening 244 of slit 232 will tend to narrow. In an exemplary embodiment of the invention, slits 226 and 228 are characterized by a width 248 of 0.03 mm, and cannula wall thickness is 0.03 mm. During plastic deformation of cannula 212, width of opening 248 increases, for example, to about 0.3 mm, for some of the slits or sections thereof (e.g., the outer portions of the slits).

In some embodiments of the invention, initial width of slits in the row along the inside of the curve produced by plastic deformation (230, 232) is larger than the initial width of the slits in the row along the outside of the curve produced by plastic deformation (226, 228). Optionally, cannula 212 may be so constructed that the larger slits (e.g. 230, 232) on the inside of the curve resulting from plastic deformation tend to close during deformation while smaller slits (e.g. 226, 228) on the inside of the curve resulting from plastic deformation tend to open or stay a same width during deformation. As a result of this construction, total cement leakage (or risk thereof) through the slits is less when cannula 212 is plastically deformed to a planned degree than it is when cannula 212 is straight.

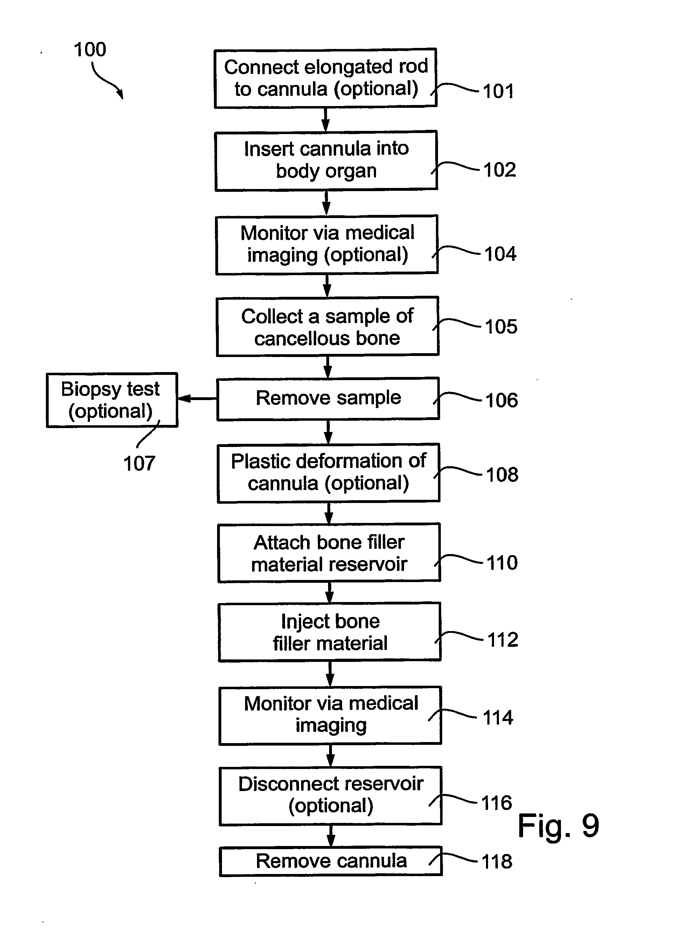

Attention is now drawn to Figure 9, which is a simplified flow chart of a method for using apparatus described hereinabove, or similar apparatus, to both remove a tissue sample from a treatment site and to inject a therapeutic material into that site. Figure 9 refers to biopsy and treatment of cancellous bone, but it is to be understood that this specific use is exemplary only, and that the apparatus and methods presented herein may be used to treat a variety of other clinical conditions.

As shown at 101, a cannula such as cannula 160 (or 300, or 212, or other cannula) may be connected to an elongated rod such as rod 600 by a interlocking mechanism such as mechanism 601 operable to join a distal side of rod 600 to a proximal side (e.g. a handle) of cannula 160. Preferably, rod 600 is long enough to extend beyond the radiation field projected by medical imaging equipment, if in use, so cannula 160 may be manually manipulated by an operator without the operator exposing his hands to the radiation field. Preferably, the cannula used has a side opening at its distal end.

At 102, a cannula (for example, a cannula 160 or 212) is inserted into a body organ, such as for example an organ having a cancellous bone. Insertion may be done with or without the aid of a stylet, which stylet may be rigid or flexible, straight or bent. Optionally, insertion 102 is monitored 104 via a medical imaging apparatus such as, for example, a fluoroscope or conventional X-ray camera.

Optionally, if required because of suspicion of a malignant tumor or other pathological condition in the body organ and/or cancellous bone, or for any other reason, a surgeon or operator may use the cannula (105) to collect a sample of cancellous bone or other tissue for pathological analysis. If this is the case, the cannula may be advanced in cancellous bone, causing a sample of cancellous bone to enter the cannula through an opening or openings at its distal end (as shown in Figure 4B), a stylet may be used to entrap and/or collect and/or remove a captured sample from the cannula (106) for optional biopsy testing (107), (as shown in Figures 4B and 4C), or biopsy sampling can be achieved by advancing a biopsy needle having an opening at its distal end until it extends beyond a distal opening of the cannula and advances thence into cancellous bone or other target tissue, causing a sample of the cancellous bone or tissue to enter into it through opening or openings at its distal end (105), as shown in Figure 4E. The biopsy needle may then be withdrawn (106), thus removing the sample from the cannula for optional biopsy testing 107.

At 108, optional plastic deformation (e.g. curving or bending) of the cannula may be performed. The cannula may be deformed before or after insertion, and, if after insertion, deformation may be performed before or after biopsy sampling.

At 110, a bone filler material reservoir and/or delivery system are attached to a proximal end of the cannula. (Other therapeutic material may be substituted instead of bone filler material.) The order in which 108 and 110 are performed is optionally reversed. In some cases, the reservoir may be integral with the cannula.

At 112, an injection of bone filler material or other flowable therapeutic material is performed, and may be monitored, either intermittently or continuously, via a medical imaging modality such as a fluoroscope or x-ray camera (114).

It is noted that plastic deformation 108 and attachment 110 may be timed or configured in view of subsequent injection monitoring 114: since a bone cement and/or injection reservoir may not be X-ray transparent, deformation 108 may be performed to configure an attached cement reservoir to be outside of a relevant portion of a field of view of X-ray imaging used during injection 112. X-rays are often obtained from a directional perpendicular to the body, e.g., along the axis of the unbent cannula, but this is not always the case and the cannula deformation may be configured accordingly. Bending of the cannula may also serve to provide convenient supports for the reservoir and other heavy items.

Injection 112 may be undertaken using a suitable injection device connected to the cement reservoir. In an exemplary embodiment of the invention, a high viscosity bone cement is employed and a high pressure injection device is employed, for example as described in "Methods, Materials and Apparatus for Treating Bone and other Tissue", cited hereinabove, the disclosure of which is incorporated herein by reference. Alternatively, a syringe or other delivery system is used. The delivered material may be, for example, PMMA or calcium-based material (such as calcium phosphate or calcium sulfate).

Optionally, a vibrator is attached to the cannula, for example, at a connector of the reservoir thereto, to facilitate flow of cement therethrough.

When injection (112) is complete as indicated by monitoring (114), the cement reservoir is disconnected (116) and the cannula is removed from the body (118). The order in which 116 and 118 are performed is optionally reversed.

Tissues which may be treated by the methods disclosed herein include, but are not limited to, the spine, compacted tibia plate and other bones with compression fractures, and bone implants such as hip implants, either during initial implanting or for tightening implants which have loosened. Optionally, for tightening an existing implant, a small hole is drilled to a location where there is a void in the bone and material is extruded into the void.

It should be noted that while the apparatus and methods presented herein provide particular advantages in treatment of bone structures, non-bone tissues such as cartilage and soft tissue may be so treated as well. The therapeutic material delivered by methods and apparatus presented herein may include an encapsulated pharmaceutical, and be used as a matrix for slow release of the pharmaceutical over time. In this manner, for example, it is possible to use means and method here provided to supply an anti-arthritis drug to a joint, by forming a void and implanting an eluting material near the joint. Such eluting material may be of high viscosity, or even be a soft non-flowing material.

In another embodiment, the injected material is a nucleus for a disc.

The following optional features are noted:

An aspect of some embodiments of the invention relates to a device, intended to serve as a channel for the delivery of material and/or instruments into the body, the device comprising a cannula and a stylet; wherein the device, when pushed through a volume containing cancellous bone, can be used to collect a sample of the cancellous bone.

Preferably, the device may be used for accessing the target organ as well. In an exemplary embodiment of the invention, the stylet and/or a cannula are introduced into a bone, for example a vertebra. In yet another exemplary embodiment of the invention, the device is introduced into the body during laparoscopic surgery.

In one embodiment of the invention, the cannula includes a side exit port. In one embodiment of the invention, the stylet has a lateral niche at it distal side. In an exemplary embodiment of the invention, the cannula and stylet can be assembled one to the other. Preferably, when stylet and cannula are assembled, the stylet niche is facing the side exit port of the cannula. In an exemplary embodiment of the invention, the cannula and stylet are assembled in at least two positions, wherein at one position the stylet niche is facing the side exit port of the cannula, and at second position, the niche is not facing the exit port. In an exemplary embodiment of the invention, the second position is met when stylet is rotated around its own axis until its niche is no longer observed through cannula's side exit port In another exemplary embodiment of the invention, the second position is met when stylet is pulled proximally at least until its niche is no longer observed through cannula's side exit port. In an alternative embodiment of the invention, the stylet has no niches or slits on its periphery which may or may not serve for any functionality such as in biopsy sampling.

Optionally, the stylet and/or cannula comprise a handle at their proximal end. Optionally, cannula and stylet are interlocked at proximal handles thereof. Optionally, there are at least two interlocking positions that permit the two assemblies positions described above. In an exemplary embodiment of the invention, the interlocking arranges the parts in a correct orientation. Optionally, the interlocking prevents inadvertent rotation or axial motion of one part relative to the other.

In an exemplary embodiment of the invention, the device is constructed from biocompatible materials. Optionally, the device is constructed from metal, such as stainless steel or a nickel-titanium alloy such as Nitinol. Optionally, at least a portion of the device is formed from a polymer.

In one embodiment of the invention, besides its side exit port, the cannula has an axial hole at its distal end suitable for introducing a stylet distal tip. Optionally, the size of the hole reduces leakage. Alternatively or additionally, the properties of the cement used reduce leakage, for example, viscosity and/or grain size. Alternatively or additionally, the cannula and/or delivery system include a plug which selectively closes the axial hole once the stylet is removed. In an alternative embodiment of the invention, the cannula has only side hole or holes at its distal end. In an exemplary embodiment of the invention, the cannula tapers at its distal end and/or is otherwise shaped, for example, to serve as a trocar.

In an exemplary embodiment of the invention, the cannula is capable of connecting to a bone filler material reservoir and/or filler material delivery system. Optionally, a proximal end of the cannula includes a connection means for attaching to a delivery device, for example a luer-lock type connector.

In one embodiment of the invention, the cannula is used to deliver bone filler material into a bone, for example a vertebral body. In one embodiment of the invention, the bone filler material is capable of setting into a hardened condition. In an exemplary embodiment of the invention, the filler is setting bone cement In one embodiment of the invention, the formulations of bone cement provide a window of time during which the cement is suitably viscous for injection. In an exemplary embodiment of the invention, the cement achieves a high viscosity rapidly when components are mixed and then sets slowly. Optionally, there is no liquid phase when cement components are mixed. In an exemplary embodiment of the invention, the bone cement has an enhanced high-viscosity window before it sets and where viscosity, while high, does not vary to a degree which influences injection parameters. In an exemplary embodiment of the invention, the cement remains viscous when held above a threshold temperature and sets when cooled below the threshold temperature. In an alternative embodiment of the invention, the bone void filler is non-setting material. Exemplary setting and non-setting bone cements are thoroughly described in PCT application PCT/IL2006/000239 and IL patent application No. 174347 by Beyar et al.

An aspect of some embodiments of the invention relates to a device, intended to serve as a channel for the delivery of material and/or instruments into the body, the device comprising a cannula, having a side opening, and a biopsy needle; wherein the device, when is located in a volume containing cancellous bone, can be used to collect a sample of the cancellous bone.

Preferably, the biopsy needle is flexible so it may bend inside a lumen of the cannula, when is pushed into the lumen, so then it may protrude out of the cannula side opening. Optionally, the biopsy needle has a bent distal side or tip so it may engage into the cannula's side opening more easily. In an exemplary embodiment of the invention, the biopsy needle is made of plastic and/or elastic and/or super-elastic, preferably bio-compatible, material. In an exemplary embodiment of the invention, the biopsy needle is made of stainless steeL

In one embodiment of the invention, the biopsy needle has an opening on its distal side. Preferably, the biopsy needle has a hollow portion directly connected to the opening, so when it protrudes out of cannula's side opening and pushed through a volume containing cancellous bone, a sample of the cancellous bone can be introduced into the opening and may be entrapped within the hollow portion. Preferably, the biopsy needle is a small diameter tube that may or may not include a handle.

The sample taken by the biopsy needle through the cannula may be removed from the biopsy needle, after the needle is withdrawn from the body, by pushing a rod through the biopsy needle, providing that the rod has an outer diameter smaller than biopsy needle inner diameter. Air pressure may also be used for this purpose.

The present invention also discloses a method for introducing bone filler material into a body organ containing a cancellous bone, the method comprising:
(a) providing a cannula having an axis and a side opening;
(b) inserting the cannula into the body organ;
(c) collecting a sample of the cancellous through the side opening;
(d) withdrawing the sample of cancellous bone out of the cannula; and
(e) injecting a flowable material through the cannula into the body organ.

In one embodiment of the invention, the cancellous bone sample is collected by inserting a biopsy needle along its axis, the biopsy needle is capable of protruding out of a cannula side opening and advancing through a cancellous bone, when sufficient force is applied. In one embodiment of the invention, the biopsy needle has an opening on its distal side. Preferably, the biopsy needle has a hollow portion directly connected to the opening, so when it protrudes out of cannula's side opening and pushed through a volume containing cancellous bone, a sample of the cancellous bone can be introduced into the opening and may be entrapped within the hollow portion. Preferably, the biopsy needle is a small diameter tube that may or may not include a handle.

In an alternative embodiment of the invention, the cancellous bone sample is collected by advancing the cannula through the cancellous bone. In one embodiment of the invention, when advancing cannula through a cancellous bone, a sample of the cancellous bone may enter into cannula inner space through its side opening. In an exemplary embodiment of the invention, the side opening is large enough and/or the travel through cancellous bone is long enough to assure that enough cancellous bone is collected for successful pathology analysis.

In one embodiment of the invention, a stylet is inserted along cannula's axis prior to insertion of cannula into the body organ. In one embodiment of the invention, the side opening of the cannula can be at least partially closed by inserting the stylet along its axis. Optionally, the stylet has a lateral niche at its distal side. In an exemplary embodiment of the invention, the cannula and stylet are assembled in at least two positions, wherein at one position the stylet niche is facing the side opening of the cannula, so side opening is at least partially opened; and when at second position, the niche is not facing the side opening, so side opening is at least partially closed with respect to first position. In an exemplary embodiment of the invention, the second position is met when stylet is rotated around its own axis. In another exemplary embodiment of the invention, the second position is met when stylet is pulled proximally. In an exemplary embodiment of the invention, after cancellous bone sample had entered the cannula's internal lumen, it may be picked up and/or taken out of the cannula by withdrawing the stylet.

An aspect of some embodiments of the invention relates to devices and/or kits for bone surgery procedures executed at least partially under fluoroscopy; the devices and/or kits include an elongated rod capable of being fixed to a bone access device and/or a filler material delivery system. In an exemplary embodiment of the invention, the elongated rod is long enough so when it is interlocked with a bone surgery instrument, and while in surgery procedure, the hands of the surgery instrument operator stays outside most or all of the radiation field projected by the fluoroscopy equipment. Preferably, the elongated rod is designed for one-time use.

In one embodiment of the invention, the elongated rod has an interlocking mechanism at its distal side. The interlocking mechanism may be based on any locking mechanism known to art and may include, though not limited to: expanding joints, flexible snaps, nut and bolt, movable teeth, forceps mechanism etc. In an exemplary embodiment of the invention, the interlocking mechanism has at least two opposing teeth on its distal side. In an exemplary embodiment of the invention, when shifting at least one of the teeth from a first position to a second position, the interlocking to a surgical instrument can occur. Preferably, the interlocking mechanism can be set to a locked position and an unlocked position by shifting the teeth. In another exemplary embodiment of the invention, the interlocking mechanism has at least two opposing flexible snaps with beveled tips which can be introduced into matching holes located in cannula proximal end and/or handle. Preferably, when the beveled tips complete a full predetermined travel through the holes, they click and interlock with the cannula. Optionally, the snaps can be pressed and/or shifted to any predetermined position in order to release the interlocking

The present invention also discloses a method for accessing into bone under fluoroscopy, the method comprising:
(a) providing a cannula having an axis, a distal side and a proximal side;
(b) providing an elongated rod having an interlocking mechanism specially designed to interlock with the proximal side of the cannula; and
(c) inserting the cannula into the body organ while holding only the elongated rod;
wherein the elongated rod is long enough so the hands of the operator holding the rod stays outside most of the radiation field projected by the fluoroscopy equipment.

Optionally, the procedure may also include delivery of bone filler material into the bone, at least in part under fluoroscopy, while holding only the elongated rod.

In one embodiment of the invention, the cannula is plastically deformable. "Plastic deformation" as used herein refers to a change in shape which requires an input energy to implement, and another energy input, for example, of similar order, to reverse. In an exemplary embodiment of the invention, the input energy required for plastic deformation is sufficiently small that it can be applied manually, optionally with one hand. Optionally, once deformed, the cannulas will not un-deform under forces applied to it by injecting cement therethrough and/or without external forces.

Optionally, plastic deformation of the cannula by hand permits a desired positioning of a cement reservoir attached thereto, relative to the cannula, imaging equipment and/or the patient. In an exemplary embodiment of the invention, insertion in a straight line is provided by the cannula in an un-deformed configuration, straight insertion being easier than insertion of a bent cannula. The inserted cannula may then be bent to a form suitable for another use, for example suitable for combined material injection and x-ray imaging.

In an exemplary embodiment of the invention, the cannula is stiff enough so that it maintains its deformation during injection of material therethrough. Optionally, the injected material is viscous, for example, viscous bone cement, injected at a high pressure, for example, at least 50 Atmospheres, or at least 100 Atmospheres, or at least 150 Atmospheres, or at least 200 Atmospheres. In an exemplary embodiment of the invention, the viscosities used are between 100 and 3000 Pascal-second, for example 300-2000, or between 500 and 1000. Intermediate values may be used as well.

In an exemplary embodiment of the invention, the cannula includes a series of joints which facilitate plastic deformation in a desired manner. In an exemplary embodiment of the invention, the joints are provided as joint areas including patterns of cuts and/or other weakening in the cannula body material. Optionally, the cuts are discrete. Optionally, the cuts are through-cuts. Alternatively, at least some of the cuts are notches or other thinning of the cannula material. Optionally, another weakening type is provided, for example, by chemical treatment and/or mechanical and/or heat treatment.

In an exemplary embodiment of the invention, the cuts and/or weakening are designed to designate particular parts of the cannula to act as plastically deformed segments, at which a significant portion of the overall deformation is provided. In an exemplary embodiment of the invention, the cuts and/or weakening are designed to facilitate a particular direction and/or degree of deformation or range of degrees. Optionally, the cuts are designed to reduce spacings in the cannula body, for example, spacings between lips of cuts.

In an exemplary embodiment of the invention, at least some of the cuts tend to at least partially close during deformation. Optionally, the tendency to partially close is related to a degree of bending the cut is subjected to. Optionally, partial closing reduces leaking of a material injected through the cannula. Optionally, the cuts are covered to reduce leaking of a material injected through the cannula. In an exemplary embodiment of the invention, a covering which substantially reduces leaking through the slits at a pressure of 100 to 200 or 300 atmospheres is provided. The covering may be, for example, internal or external, for example, of Teflon.

Optionally, more viscous cement is used with the cannula, to reduce leakage. In an exemplary embodiment of the invention, a cement is selected which reduces leakage in other means, for example, the cement may include a liquid phase and a solid phase, with the solid particles being of the order of the narrow dimensions of the slits or larger. Optionally, leakage is reduced once the solid particles are at least 10%, at least 35%, at least 60%, at least 80% or larger of such narrow dimensions, for example, being at least 0.01 mm, 0.05 mm, 0.1 mm, 0.3 mm, or intermediate or greater in size.

In an exemplary embodiment of the invention, the joints each comprise an asymmetric cut design, in which a greater spacing between lips is provided on one side of the cut, so that bending in the direction of that side, tends to reduce the spacing.

In an exemplary embodiment of the invention, the joints are arranged in a line parallel to the cannula axis.

In an exemplary embodiment of the invention, each joint permits a deformation of 5, optionally 10, optionally 15 degrees or lesser or greater or intermediate values. Optionally, a total deformation of 45, optionally 90, optionally 135, optionally 180 degrees or smaller, intermediate values or greater is achieved and/or is designated as a design set point where there is minimal leakage. The length of cannula subject to deformation may vary with the total deformation implemented and/or the number of slits employed, for example, being 20 mm, 30 mm, 40 mm, or greater, smaller or intermediate in size. In an exemplary embodiment of the invention, 13 cuts provide a total deformation of 130 degrees. Greater or smaller numbers of cuts/deformation regions may be provided, for example, 7, 10, 15 or smaller, intermediate or greater numbers.

It will be appreciated that the above described apparatus and methods of implanting and treating may be varied in many ways, including, changing the order of steps, selecting which steps are performed more often and which less often, arrangements of elements, type and magnitude of forces applied and/or particular shapes used. In particular, various tradeoffs may be desirable, for example, between applied forces, degree of resistance and forces that can be withstood by treated organs. Further, the location of various elements may be switched without departing from the spirit of the disclosure, For example, location of the cement outlet may be displaced within the cannula to suit convenience or the specific geometry of a particular treatment target.

It is further noted that a multiplicity of features, both of method and of apparatus, have been described. It should be appreciated that various features may be combined in different ways, and that not all features shown herein in the context of particular exemplar embodiments are necessary in all other embodiment of the invention. Further, combinations of presented features, which combinations are not explicitly presented herein, are also to be considered to be within the scope of the invention.

In addition, some of the features of the invention described herein may be adapted for use with prior art devices. The particular geometric forms used to illustrate the invention should not be considered limiting. For example, where a cylindrical tube is shown, in other embodiments a rectangular tube may be used. The scope of the invention also includes apparatus programmed and/or designed to carry out the methods presented herein.

Measurements are provided to serve only as exemplary measurements for particular cases, the exact measurements applied will vary depending on the application.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art.

## Claims

1. An apparatus (150) for extracting a biopsy sample and for injecting a therapeutic material, comprising:
(a) a cannula (160; 212; 300; 700; 800) having an internal lumen (166) and a lateral distal opening (162; 301; 801);
(b) a tissue extractor (805) having a distal portion able to traverse said lumen, and advance through said lateral distal opening to receive and hold a biopsy specimen; and
(c) an injector (190) connectable to said cannula and operable to inject a flowable material therethrough.

2. The apparatus of claim 1, wherein said lateral distal opening (162) is adapted for collecting a sample of cancellous bone tissue when said cannula is inserted in a bone.

3. The apparatus of claim 1, wherein said cannula (212) is designed to undergo plastic deformation after insertion of a distal portion of said cannula into a body, and further comprising a plurality of slits (224) which facilitate plastic deformation of said cannula.

4. The apparatus of claim 1, further comprising a stylet (214) advancable and retractable within said cannula and configured to stiffen said cannula.

5. The apparatus of claim 1, further comprising a stylet advancable and retractable within said cannula, said stylet being configured to cover the lateral distal opening of the cannula.

6. The apparatus of claim 5, wherein the appearance of said stylet tip is of visibly different radio-opacity from distal portions of said cannula, when both are viewed by means of a clinical visual modality.

7. The apparatus of claim 5, wherein said stylet tip is of visibly different diameter from the distal portions of said cannula, when both are viewed by means of a clinical visual modality.

8. The apparatus of claim 1, wherein a surface feature of said stylet and a corresponding surface feature of said lumen of said cannula combine to constrain said stylet to enter said cannula in a predetermined orientation.

9. The apparatus of claim 1, wherein said tissue extractor comprises a biopsy needle (805) having a flexible distal portion, said flexible distal portion extending from said lateral opening when said biopsy needle is fully inserted in said cannula.

10. The apparatus of claim 9, wherein a distal end (815) of an internal lumen (814) of said cannula slants in a direction which forces said biopsy needle towards said lateral opening when said biopsy needle is forced against said distal end of said internal lumen of said cannula.

11. The apparatus of claim 1, wherein said injector comprises a reservoir (192) of flowable material.

12. The apparatus of claim 1, further comprising a vibrator (193).

13. The apparatus of claim 1, wherein said cannula has an open distal tip.

14. The apparatus of claim 1, wherein said cannula has a closed distal tip.

15. The apparatus of claim 1, wherein said lateral distal opening is adapted for collecting a sample of cancellous bone when said cannula is inserted in bone.

16. The apparatus of claim 1, wherein said flowable material comprises bone filler material.

17. The apparatus of claim 1, wherein said flowable material comprises high viscosity bone filler.

18. The apparatus of claim 1, wherein said flowable material comprises bone cement.

19. The apparatus of claim 1, wherein said flowable material comprises bone cement having a viscosity in a range of about 100 to 3000 Pascal-seconds.

20. The apparatus according to any of claims 1 to 19, wherein said injector further comprises a pump configured to inject said flowable material at a pressure of 50 atmospheres to 100 atmospheres.

## Patentansprüche

1. Gerät (150) zum Extrahieren einer Biopsieprobe und zum Injizieren eines therapeutischen Material, umfassend:
(a) eine Kanüle (106; 212; 200; 700; 800) mit einem inneren Lumen (166) und einer lateralen distalen Öffnung (162; 301; 801);
(b) einem Gewebeextraktor (805) mit einem distalen Abschnitt, der fähig ist, das Lumen zu durchqueren und sich durch die laterale distale Öffnung zum Aufnehmen und Halten einer Biopsieprobe zu bewegen; und
(c) einen Injektor (190), der mit der Kanüle verbindbar und betreibbar ist, um dort hindurch ein fließfähiges Material zu injizieren.

2. Gerät nach Anspruch 1, wobei die laterale distale Öffnung (162) zum Sammeln einer Probe von Knochenspongiosagewebe bei Einführung der Kanüle in einen Knochen gestaltet ist.

3. Gerät nach Anspruch 1, wobei die Kanüle (212) entworfen ist, um einer plastischen Verformung nach Einführen eines distalen Abschnitts der Kanüle in einen Körper unterzogen zu werden, und ferner eine Vielzahl von Schlitzen (224) aufweist, die eine plastische Verformung der Kanüle erleichtern.

4. Gerät nach Anspruch 1, ferner umfassend ein Stilett (214), das in der Kanüle vorschiebbar und einschiebbar und zum Versteifen der Kanüle konfiguriert ist.

5. Gerät nach Anspruch 1, ferner umfassend ein in der Kanüle vorschiebbares und einschiebbares Stilett, wobei das Stilett konfiguriert ist, um die laterale distale Öffnung der Kanüle abzudecken.

6. Gerät nach Anspruch 5, wobei die Erscheinung der Stilettspitze eine optisch andere Strahlenundurchlässigkeit gegenüber distalen Abschnitten der Kanüle aufweist, wenn beide mittels eines klinischen optischen Verfahrens betrachtet werden.

7. Gerät nach Anspruch 5, wobei die Stilettspitze einen optisch erkennbar anderen Durchmesser gegenüber den distalen Abschnitten der Kanüle aufweist, wenn beide mittels eines klinischen optischen Verfahrens beobachtet werden.

8. Gerät nach Anspruch 1, wobei ein Oberflächenmerkmal des Stiletts und ein korrespondierendes Oberflächenmerkmal des Lumens der Kanüle sich verbinden, um das Stilett dazu zu zwingen, in einer vorab festgelegten Orientierung in die Kanüle einzutreten.

9. Gerät nach Anspruch 1, wobei der Gewebeextraktor eine Biopsienadel (8ß5) mit einem flexiblen distalen Abschnitt aufweist, wobei sich der flexible distale Abschnitt von der lateralen Öffnung erstreckt, wenn die Biopsienadel in die Kanüle vollständig eingeführt ist.

10. Gerät nach Anspruch 9, wobei ein distales Ende (815) eines inneren Lumens (814) der Kanüle in einer Richtung abgeschrägt ist, die die Biopsienadel in Richtung zur lateralen Öffnung zwingt, wenn die Biopsienadel gegen das distale Ende des inneren Lumens der Kanüle gezwungen wird.

11. Gerät nach Anspruch 1, wobei der Injektor einen Behälter (192) mit fließfähigem Material aufweist.

12. Gerät nach Anspruch 1, ferner umfassend einen Schwingungserzeuger (193).

13. Gerät nach Anspruch 1, wobei die Kanüle eine offene distale Spitze aufweist.

14. Gerät nach Anspruch 1, wobei die Kanüle eine geschlossene distale Spitze aufweist.

15. Gerät nach Anspruch 1, wobei die laterale distale Öffnung zum Sammeln einer Probe von Knochenspongiosa bei Einführung der Kanüle in Knochen gestaltet ist.

16. Gerät nach Anspruch 1, wobei das fließfähige Material Knochenersatzmaterial umfasst.

17. Gerät nach Anspruch 1, wobei das fließfähige Material hoch viskoses Knochenersatzmaterial umfasst.

18. Gerät nach Anspruch 1, wobei das fließfähige Material Knochenzement umfasst.

19. Gerät nach Anspruch 1, wobei das fließfähige Material Knochenzement mit einer Viskosität in einem Bereich von ca. 100 bis 3000 Pascal-Sekunde umfasst.

20. Gerät nach einem der Ansprüche 1 bis 19, wobei der Injektor ferner eine Pumpe umfasst, die zum Injizieren des fließfähigen Material mit einem Druck von 50 Atmosphären bis 100 Atmosphären konfiguriert ist.

## Revendications

1. Appareil (150) pour extraire un échantillon de biopsie et pour injecter un matériau thérapeutique, comprenant :
(a) une canule (160 ; 212 ; 300 ; 700 ; 800) ayant une lumière interne (166) et une ouverture distale latérale (162 ; 301 ; 801) ;
(b) un extracteur de tissu (805) ayant une portion distale apte à traverser ladite lumière et à avancer à travers ladite ouverture distale latérale pour recevoir et retenir un spécimen de biopsie ; et
(c) un injecteur (190) pouvant être connecté à ladite canule et apte à injecter un matériau coulant à travers celle-ci.

2. Appareil selon la revendication 1, dans lequel ladite ouverture distale latérale (162) est apte à recueillir un échantillon de tissu d'os spongieux lorsque ladite canule est insérée dans un os.

3. Appareil selon la revendication 1, dans lequel ladite canule (212) est conçue pour subir une déformation plastique après l'insertion d'une portion distale de ladite canule dans un corps, et comprenant en outre une pluralité de fentes (224) qui facilitent la déformation plastique de ladite canule.

4. Appareil selon la revendication 1, comprenant en outre un stylet (214) apte à avancer et à se rétracter dans ladite canule et configuré pour rendre rigide ladite canule.

5. Appareil selon la revendication 1, comprenant en outre un stylet apte à avancer et à se rétracter dans ladite canule, ledit stylet étant configuré pour couvrir l'ouverture distale latérale de la canule.

6. Appareil selon la revendication 5, dans lequel l'aspect de ladite pointe de stylet est d'une radio-opacité visiblement différente des portions distales de ladite canule, lorsque les deux sont vues au moyen d'une modalité visuelle clinique.

7. Appareil selon la revendication 5, dans lequel ladite pointe du stylet a un diamètre visiblement différent des portions distales de ladite canule, lorsque les deux sont vues au moyen d'une modalité visuelle clinique.

8. Appareil selon la revendication 1, dans lequel une propriété de surface dudit stylet et une propriété de surface correspondante de ladite lumière de ladite canule se combinent pour contraindre ledit stylet à entrer dans ladite canule selon une orientation prédéterminée.

9. Appareil selon la revendication 1, dans lequel ledit extracteur de tissu comprend une aiguille de biopsie (805) comportant une portion distale flexible, ladite portion distale flexible s'étendant de ladite ouverture latérale lorsque ladite aiguille de biopsie est entièrement insérée dans ladite canule.

10. Appareil selon la revendication 9, dans lequel une extrémité distale (815) d'une lumière interne (814) de ladite canule est inclinée dans une direction qui force ladite aiguille de biopsie vers ladite ouverture latérale lorsque ladite aiguille de biopsie est forcée contre ladite extrémité distale de ladite lumière interne de ladite canule.

11. Appareil selon la revendication 1, dans lequel ledit injecteur comprend un réservoir (192) de matériau coulant.

12. Appareil selon la revendication 1, comprenant en outre un vibreur (193).

13. Appareil selon la revendication 1, dans lequel ladite canule comporte une pointe distale ouverte.

14. Appareil selon la revendication 1, dans lequel ladite canule possède une pointe distale fermée.

15. Appareil selon la revendication 1, dans lequel ladite ouverture distale latérale est apte à recueillir un échantillon d'os spongieux lorsque ladite canule est insérée dans l'os.

16. Appareil selon la revendication 1, dans lequel ledit matériau coulant comprend un matériau de comblement osseux.

17. Appareil selon la revendication 1, dans lequel ledit matériau coulant comprend un comblement osseux hautement visqueux.

18. Appareil selon la revendication 1, dans lequel ledit matériau coulant comprend un ciment d'os.

19. Appareil selon la revendication 1, dans lequel ledit matériau coulant comprend un ciment d'os d'une viscosité dans une plage d'environ 100 à 3000 Pascal secondes.

20. Appareil selon l'une quelconque des revendications 1 à 19, dans lequel ledit injecteur comprend en outre une pompe configurée pour injecter ledit matériau coulant à une pression de 20 atmosphères à 100 atmosphères.
